# FASCICULE DE BREVET EUROPEEN

(11) **EP 3 027 588 B1**
(45) Date de publication et mention de la délivrance du brevet: **01.04.2020**
(21) Numéro de dépôt: 14758162.3
(22) Date de dépôt: 28.07.2014
(51) Int. Cl.: C07C 303/32, C07C 233/18

(54) **COMPLEXES D'AGOMÉLATINE ET D'ACIDES SULFONIQUES, PROCÉDÉ DE LEUR PRÉPARATION ET COMPOSITIONS PHARMACEUTIQUES QUI LES CONTIENNENT**
KOMPLEXE VON AGOMELATIN UND SULFONSÄUREN, VERFAHREN ZU DEREN HERSTELLUNG UND DIESE ENTHALTENDE PHARMAZEUTISCHE ZUSAMMENSETZUNGEN
COMPLEXES OF AGOMELATINE AND SULPHONIC ACIDS, METHOD FOR PREPARING SAME AND PHARMACEUTICAL COMPOSITIONS CONTAINING THEM

(30) Priorité: 29.07.2013 WO PCT/CN2013/080337; 17.10.2013 FR 1360124
(43) Date de publication de la demande: 08.06.2016
(73) Titulaire: Les Laboratoires Servier, 92284 Suresnes (FR); Shanghai Institute Of Pharmaceutical Industry, Shanghai 200040 (CN)
(72) Inventeur: SHAN, Hanbin, Gaoan Jiangxi 330800 (CN); SHEN, Yuhui, Shanghai 200439 (CN); LUO, Ying, Nan Chang Jiangxi 300006 (CN); LETELLIER, Philippe, 45000 Orléans (FR); LYNCH, Michael, 45140 Saint Jean de la Ruelle (FR)
(74) Mandataire: Bestel, Delphine
(86) Numéro de dépôt international: PCT/FR2014/051944
(87) Numéro de publication internationale: WO 2015/015102

(56) Documents cités:
- EP-A1- 2 517 700
- WO-A1-2013/170738

## Description

La présente invention concerne de nouveaux complexes d'agomélatine et d'acides sulfoniques, leur procédé de préparation ainsi que les compositions pharmaceutiques qui les contiennent.

L'agomélatine ou *N*-[2-(7-méthoxy-1-naphtyl)éthyl]acétamide a la structure de formule (II) :

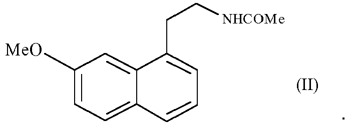

L'agomélatine est commercialisé sous le nom de marque Valdoxan® ou Thymanax® par le groupe français Servier en tant qu'agoniste sur les récepteurs du système mélatoninergique et antagoniste du récepteur 5-HT_{2C}. C'est le premier antidépresseur de type mélatoninergique, utile dans le traitement de la dépression majeure, améliorant le sommeil et la fonction sexuelle.

L'agomélatine, sa préparation et son utilisation en thérapeutique ont été décrits dans les brevets européens EP 0 447 285 et EP1564202.

Le brevet européen EP2517700 décrit des co-cristaux d'agomélatine avec l'acide citrique, l'acide benzènesulfonique et l'acide maléique de stoechiométrie 1/1.

L'objet de la présente invention consiste en l'élaboration de complexes d'agomélatine et d'acides sulfoniques ayant la stœchiométrie particulière de 2 équivalents molaires d'agomélatine pour 1 équivalent molaire d'acides sulfoniques. Ces complexes présentent d'excellentes propriétés en matière de solubilité, stabilité et pureté, permettant d'envisager leur utilisation pour la fabrication de compositions pharmaceutiques contenant de l'agomélatine. Par ailleurs, la stœchiométrie des complexes selon la présente invention confère un avantage pondéral en faveur du principe actif du complexe *i.e.* l'agomelatine, permettant d'élaborer des formulations pharmaceutiques contenant de plus faibles quantités de complexe.

La présente invention concerne des complexes d'agomélatine et d'acides sulfoniques ayant la structure de formule (I) : 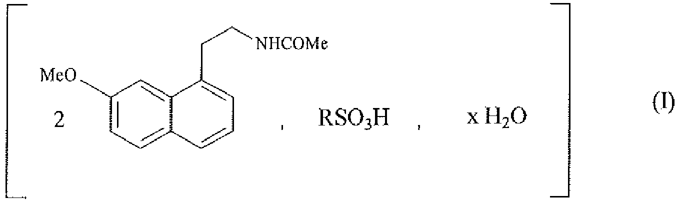 dans laquelle x représente 0 ou 1, et RSO₃H représente l'acide 1,5-naphtalène disulfonique ou l'acide benzène sulfonique.

Les composés préférés selon l'invention sont les complexes d'agomélatine et d'acides sulfoniques suivants :
- complexe agomélatine / acide 1,5-naphtalène disulfonique (2/1),
- complexe agomélatine / acide 1,5-naphthalène disulfonique (2/1) monohydrate,
- complexe agomélatine / acide benzène sulfonique (2/1).

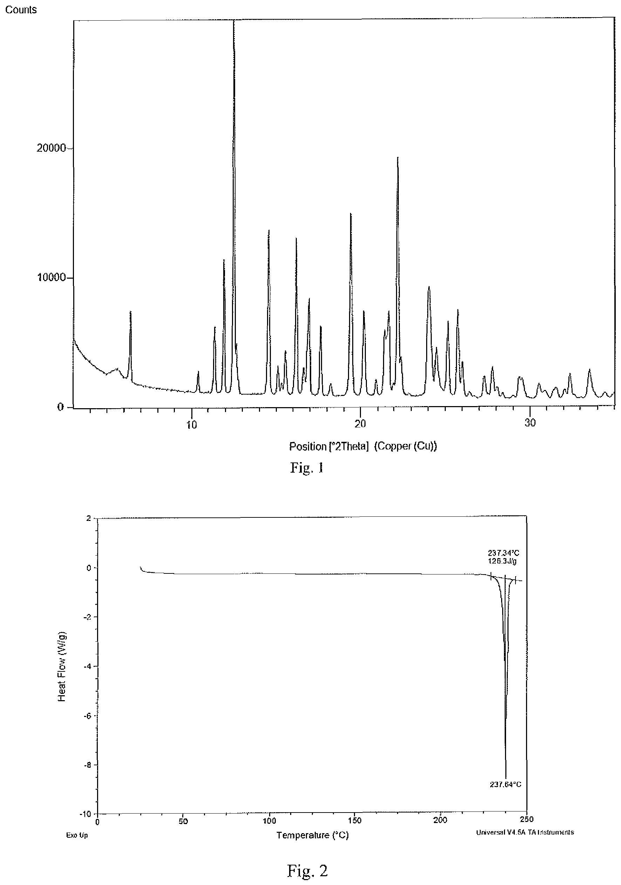

Le complexe agomélatine / acide 1,5-naphtalène disulfonique (2/1) est caractérisé par son diagramme de diffraction X sur poudre rprésenté dans la Figure 1, mesuré sur un diffractomètre Panalytical Xpert Pro MPD (anticathode de cuivre). Les raies principales sont exprimées en termes de distance inter-réticulaire d, d'angle de Bragg 2 thêta (exprimés en °±0,2), et d'intensité relative (exprimée en pourcentage par rapport à la raie la plus intense) et sont listées dans le Tableau 1:

**Tableau 1: Tableau des pics de diffraction du complexe agomélatine / acide 1,5-naphtalène disulfonique (2/1)**

| 2-Theta (°) exp. | d (Å) exp. | Intensité (%) |
|---|---|---|
| 6,3716 | 13,87229 | 18,97 |
| 11,3804 | 7,77552 | 17,98 |
| 11,9227 | 7,42299 | 36,06 |
| 12,5064 | 7,07784 | 100,00 |
| 12,6590 | 6,99288 | 13,75 |
| 14,5508 | 6,08767 | 44,17 |
| 15,5658 | 5,69292 | 11,96 |
| 16,2029 | 5,47051 | 42,63 |
| 16,9421 | 5,23346 | 25,85 |
| 17,6267 | 5,03171 | 18,67 |
| 19,4300 | 4,56857 | 49,04 |
| 20,2146 | 4,39301 | 22,77 |
| 21,4353 | 4,14550 | 17,80 |
| 21,6713 | 4,10090 | 22,84 |
| 22,2180 | 4,00121 | 64,19 |
| 22,4174 | 3,96607 | 10,83 |
| 24,0749 | 3,69664 | 29,61 |
| 24,5048 | 3,63275 | 13,33 |
| 25,1744 | 3,53763 | 20,58 |
| 25,7599 | 3,45853 | 23,59 |

Lorsque le complexe de la présente invention est caractérisé par la mesure de diffraction aux rayons X, il peut y avoir des erreurs de mesure des pics identifiés parfois attribuables à l'équipement ou aux conditions utilisées. Plus particulièrement, les valeurs 2 thêta peuvent avoir une erreur d'environ ± 0,2, et parfois une erreur d'environ ± 0,1 même si des équipements de haute technicité sont utilisés. Ainsi, l'erreur de mesure doit être prise en compte lors de l'identification de la structure du complexe.

La structure cristalline du complexe agomélatine / acide 1,5-naphtalène disulfonique (2/1) a été déterminée et les paramètres suivants ont été identifiés:
- Groupe d'espace : P 1 21/c 1 (14)
- Paramètres de maille: a = 8,4970(3)Â, b = 8,0873(3)Å, c = 27,7107(9)Å; α = 90°, β = 93.059(2)°, γ = 90°
- Volume de la maille : V_{unit cell} = 1901,51100Å³

Le complexe agomélatine / acide 1,5-naphtalène disulfonique (2/1) est également caractérisé par DSC (differential scanning calorimetry) dans le spectre représenté dans la Figure 2, qui montre un endotherme correspondant à la fusion du complexe à une température d'environ 237°C.

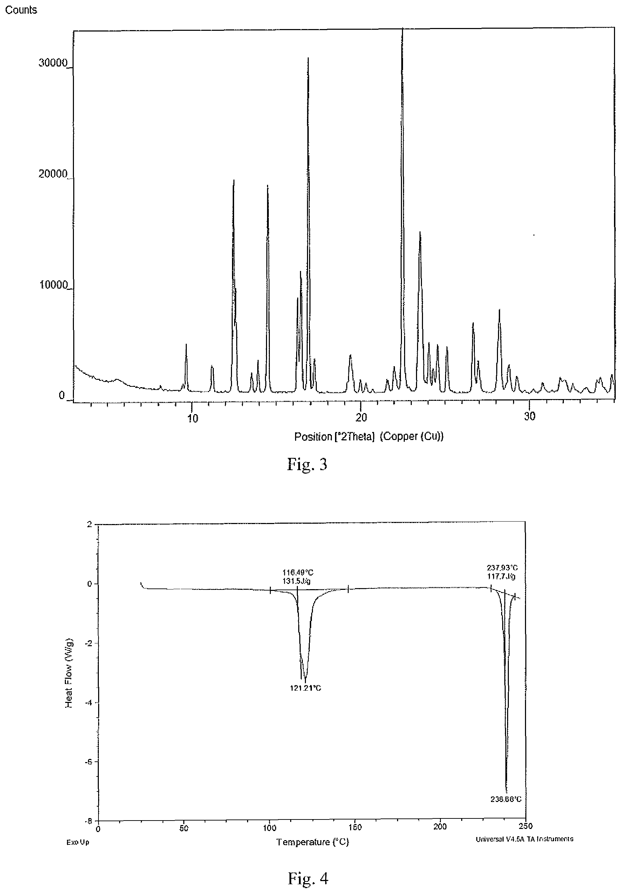

L'invention concerne également le complexe agomélatine / acide 1,5-naphthalène disulfonique (2/1) monohydrate qui est caractérisé par son diagramme de diffraction X sur poudre représenté dans la Figure 3, mesuré sur un diffractomètre Panalytical Xpert Pro MPD (anticathode de cuivre). Les raies principales sont exprimées en termes de distance inter-réticulaire d, d'angle de Bragg 2 thêta (exprimés en °±0,2), et d'intensité relative (exprimée en pourcentage par rapport à la raie la plus intense) et sont listées dans le Tableau 2:

**Tableau 2: Tableau des pics de diffraction du complexe agomélatine / acide 1,5-naphthalène disulfonique (2/1) monohydrate**

| 2-Theta (°) exp. | d (Å) exp. | Intensité (%) |
|---|---|---|
| 9,6680 | 9,14852 | 12,45 |
| 12,4885 | 7,08796 | 57,23 |
| 12,6164 | 7,01639 | 28,61 |
| 14,5042 | 6,10715 | 57,42 |
| 16,2684 | 5,44863 | 25,67 |
| 16,4624 | 5,38484 | 32,93 |
| 16,8967 | 5,24739 | 90,90 |
| 19,3772 | 4,58091 | 10,50 |
| 22,4767 | 3,95573 | 100,00 |
| 23,4111 | 3,79992 | 20,49 |
| 23,5330 | 3,78051 | 44,02 |
| 23,6735 | 3,75840 | 21,92 |
| 24,0477 | 3,70076 | 13,67 |
| 24,5716 | 3,62303 | 13,43 |
| 25,1240 | 3,54460 | 12,46 |
| 26,6602 | 3,34374 | 19,10 |
| 28,1333 | 3,16930 | 12,07 |
| 28,2443 | 3,15971 | 22,49 |

Lorsque le complexe de la présente invention est caractérisé par la mesure de diffraction aux rayons X, il peut y avoir des erreurs de mesure des pics identifiés parfois attribuables à l'équipement ou aux conditions utilisées. Plus particulièrement, les valeurs 2 thêta peuvent avoir une erreur d'environ ± 0,2, et parfois une erreur d'environ ± 0,1 même si des équipements de haute technicité sont utilisés. Ainsi, l'erreur de mesure doit être prise en compte lors de l'identification de la structure du complexe.

La structure cristalline du complexe agomélatine / acide 1,5-naphtalène disulfonique (2/1) monohydrate a été déterminée et les paramètres suivants ont été identifiés:
- Groupe d'espace : P -1 (2)
- Paramètres de maille: a = 9,5673(3) Å, b = 9,7223(3) Å, c = 11,4632(3) Å; α = 76,967(2)°, β = 75,339(1)°, γ = 78,675(2)°
- Volume de la maille : V_{unit cell} = 993,93800 Å³

Le complexe agomélatine / acide 1,5-naphtalène disulfonique (2/1) monohydrate est également caractérisé par DSC (differential scanning calorimetry) dans le spectre représenté dans la Figure 4, qui montre deux endothermes : l'un à environ 116°C correspondant à la déshydratation du complexe, l'autre à environ 238°C correspondant à la fusion du complexe.

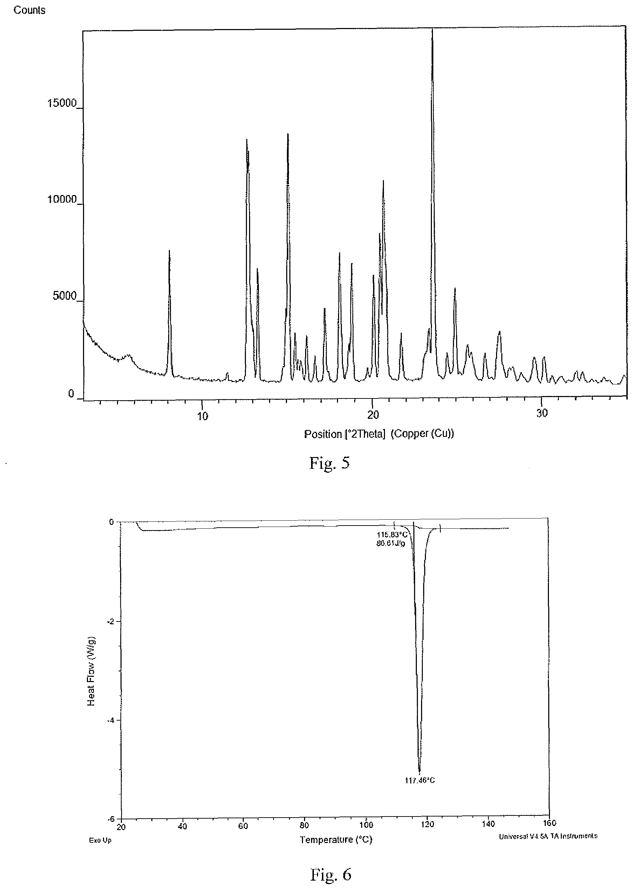

L'invention concerne également le complexe agomélatine / acide benzène sulfonique (2/1) qui est caractérisé par son diagramme de diffraction X sur poudre représenté dans la Figure 5, mesuré sur un diffractomètre Panalytical Xpert Pro MPD (anticathode de cuivre). Les raies principales sont exprimées en termes de distance inter-réticulaire d, d'angle de Bragg 2 thêta (exprimés en °±0,2), et d'intensité relative (exprimée en pourcentage par rapport à la raie la plus intense) et sont listées dans le Tableau 3:

**Tableau 3: Tableau des pics de diffraction du complexe agomélatine / acide benzène sulfonique (2/1)**

| 2-Theta (°) exp. | d (Å) exp. | Intensité (%) |
|---|---|---|
| 8,0711 | 10,95469 | 35,25 |
| 12,6820 | 6,98026 | 68,37 |
| 12,7706 | 6,93203 | 65,37 |
| 13,0114 | 6,80427 | 15,18 |
| 13,3054 | 6,65458 | 31,84 |
| 14,9475 | 5,92700 | 19,42 |
| 15,1121 | 5,86283 | 70,19 |
| 15,4873 | 5,72160 | 14,16 |
| 16,1644 | 5,48344 | 12,98 |
| 17,2360 | 5,14486 | 21,06 |
| 18,1046 | 4,89993 | 36,33 |
| 18,6255 | 4,76406 | 10,91 |
| 18,8009 | 4,72001 | 33,43 |
| 20,0908 | 4,41978 | 30,26 |
| 20,4742 | 4,33788 | 42,37 |
| 20,6921 | 4,29270 | 56,78 |
| 20,8640 | 4,25771 | 26,42 |
| 21,7142 | 4,09289 | 13,88 |
| 23,3683 | 3,80679 | 15,16 |
| 23,6410 | 3,76349 | 100,00 |
| 24,9314 | 3,57154 | 26,81 |
| 25,6543 | 3,47253 | 10,71 |
| 27,5599 | 3,23660 | 14,00 |

Lorsque le complexe de la présente invention est caractérisé par la mesure de diffraction aux rayons X, il peut y avoir des erreurs de mesure des pics identifiés parfois attribuables à l'équipement ou aux conditions utilisées. Plus particulièrement, les valeurs 2 thêta peuvent avoir une erreur d'environ ± 0,2, et parfois une erreur d'environ ± 0,1 même si des équipements de haute technicité sont utilisés. Ainsi, l'erreur de mesure doit être prise en compte lors de l'identification de la structure du complexe.

La structure cristalline du complexe agomélatine / acide benzène sulfonique (2/1) a été déterminée et les paramètres suivants ont été identifiés:
- Groupe d'espace : P -1 (2)
- Paramètres de maille: a = 15,5878(8) Å, b = 15,7088(6) Å, c = 7,2091(3) Å; α = 100,445(2)°, β = 99,470(2)°, γ = 89,054(3)°
- Volume de la maille : V_{unit cell} = 1712,18900 Å

Le complexe agomélatine / acide benzène sulfonique (2/1) est également caractérisé par DSC (differential scanning calorimetry) dans le spectre représenté dans la Figure 6, qui montre un endotherme à environ 116°C correspondant à la fusion du complexe.

L'invention concerne également un procédé d'obtention des complexes d'agomélatine et d'acides sulfoniques, dans lequel :
- les deux constituants sont mélangés au sein d'un solvant organique ou hydro-organique dans les proportions désirées;
- la solution obtenue est agitée et optionnellement chauffée à une température au plus égale à la température d'ébullition du solvant choisi ;
- le milieu est refroidi sous agitation et le complexe précipite naturellement ou précipite après reprise dans un deuxième solvant ;
- le précipité obtenu est filtré et séché.

Dans le procédé selon l'invention, le solvant utilisé est préférentiellement une cétone comme par exemple l'acétone ; un éther comme par exemple l'éther diisopropylique, le tétrahydrofurane ou l'éther de méthyle et de *tert*-butyle ; un hydrocarbure aromatique comme le toluène par exemple. Lorsqu'un deuxième solvant est utilisé pour favoriser la précipitation du complexe, le solvant choisi est un alcool comme par exemple le méthanol, l'éthanol ou le *tert*-butanol ; un alkane comme par exemple le *n*-hexane ou le *n*-heptane ; ou le benzonitrile.

Un procédé alternatif consiste à co-broyer les deux constituants du co-cristal. Préférentiellement le co-broyage est effectué dans une jarre en acier. Une variante de ce procédé consiste à rajouter un solvant organique lors du broyage ; dans ce cas le co-cristal obtenu est ensuite séché. Parmi les solvants utilisés on citera plus particulièrement les cétones comme par exemple l'acétone ; ou les éthers comme par exemple l'éther diisopropylique, ou l'éther de méthyle et de *tert*-butyle. Les alcools comme par exemple le méthanol l'éthanol ou le tert-butanol peuvent également être utilisés.

Avantageusement, le broyage est réalisé avec des billes en inox. Le broyage est réalisé au moyen de vibrations, préférentiellement des vibrations avec une fréquence allant de 20 à 30 Hz. Les vibrations sont appliquées pendant une durée pouvant aller de 5 minutes à 3 heures.

Un autre procédé alternatif consiste à mélanger deux solutions contenant chacun des constituants et à congeler rapidement à très basse température le mélange obtenu, puis à sécher à cette même basse température le co-cristal ainsi obtenu. Avantageusement, les deux constituants sont mélangés au sein d'un solvant organique ou hydro-organique. De façon préférée, la congélation et le séchage sont effectués entre -40°C et -60°C, et plus préférentiellement à -40°C.

Un autre procédé avantageux selon l'invention consiste à mélanger les poudres d'agomélatine et de l'acide considéré dans un mélangeur, puis à l'extruder par extrusion bi-vis sans filière pour obtenir un grain solide directement en sortie d'extrudeuse. De préférence, le profil de vis utilisé est un profil à fort cisaillement, avec optionnellement l'utilisation d'éléments malaxeurs permettant d'améliorer la surface de contact entre les constituants. Le paramètre L/D de la vis peut varier entre 10 et 40 et la vitesse de rotation entre 10 et 200 tr/min. La température utilisée varie de 40 à 100 °C.

Les complexes d'agomélatine et d'acides sulfoniques obtenus ont une solubilité accrue de façon très significative par rapport à l'agomélatine *per se,* ce qui les rend plus appropriés pour l'élaboration de formulations pharmaceutiques. Les complexes d'agomélatine et d'acides sulfoniques selon l'invention présentent de plus une excellente stabilité et une très bonne pureté. Ils sont par ailleurs obtenus par un procédé simple, ne comportant aucune étape difficile.

Les formes pharmaceutiques contenant les complexes selon l'invention seront utilisées pour le traitement des désordres du système mélatoninergique, et plus particulièrement dans le traitement du stress, des troubles du sommeil, des troubles de l'anxiété et notamment du trouble anxiété généralisée, des troubles obsessionnels compulsifs, des troubles de l'humeur et notamment des troubles bipolaires, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, les co-cristaux selon l'invention pourront être utilisés dans les dysfonctionnements sexuels, en tant qu'inhibiteurs de l'ovulation, immunomodulateurs et dans le traitement des cancers.

L'invention s'étend aussi aux compositions pharmaceutiques renfermant comme principe actif un complexe d'agomélatine et d'acides sulfoniques selon l'invention avec un ou plusieurs adjuvants ou excipients.

Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les granulés, les comprimés sublinguaux, les gélules, les tablettes, les suppositoires, les crèmes, les pommades, les gels dermiques, les préparations injectables, les suspensions buvables et les pâtes à mâcher.

La posologie utile est adaptable selon la nature et la sévérité de l'affection, la voie d'administration ainsi que l'âge et le poids du patient. Cette posologie varie de 0,1 mg à 1 g par jour d'agomélatine en une ou plusieurs prises.

Des exemples représentatifs de la présente invention sont illustrés avec les Figures correspondantes afin de mieux en apprécier l'objet, les caractéristiques, et les avantages.
Figure 1 : diagramme de diffraction X sur poudre du complexe agomélatine / acide 1,5-naphtalène disulfonique (2/1) de l'Exemple 1.
Figure 2: thermogramme DSC du complexe agomélatine / acide 1,5-naphtalène disulfonique (2/1) de l'Exemple 1.
Figure 3 : diagramme de diffraction X sur poudre du complexe agomélatine / acide 1,5-naphtalène disulfonique (2/1) monohydrate de l'Exemple 2.
Figure 4 : thermogramme DSC du complexe agomélatine / acide 1,5-naphtalène disulfonique (2/1) monohydrate de l'Exemple 2.
Figure 5 : diagramme de diffraction X sur poudre du complexe agomélatine / acide benzène sulfonique (2/1) de l'Exemple 3.
Figure 6 : thermogramme DSC du complexe agomélatine / acide benzène sulfonique (2/1) de l'Exemple 3.

### Exemple 1 : Complexe agomélatine / acide 1,5-naphtalène disulfonique (2/1)

### Mode-opératoire 1

L'agomélatine (5,00g, 2 eq) et l'acide 1,5-naphtalène disulfonique anhydre (2,96g, 1 eq) sont placés dans un réacteur. 20ml d'acétone sont ajoutés. La suspension est agitée sous reflux pendant 1 heure, puis immédiatement filtrée. Le gâteau est lavé 2 fois à l'acétone, puis séché pendant 1 heure. 25g d'un solide blanc correspondant au produit du titre sont obtenus.
Rendement: 78,5%
Point de fusion : 237°C

### Mode-opératoire 2

L'agomélatine (2,98g, 2 eq) et l'acide 1,5-naphtalène disulfonique tétrahydrate (2,18g, 1 eq) sont introduits dans un ballon de 250 ml. 100 ml d'acétone sont ajoutés et le milieu réactionnel est porté au reflux pendant 3 heures (la cristallisation se produit au bout d'une heure environ). La suspension est refroidie à température ambiante et agitée pendant 1 heure. 4,03g d'un solide blanc correspondant au produit du titre sont isolés par filtration et séchés sous vide (10 mbars) à 40°C pendant 15 heures.
Rendement : 85,0%
Point de fusion : 237°C

### Mode-opératoire 3

L'agomélatine (5,00g, 2 eq) et l'acide 1,5-naphtalène disulfonique anhydre (2,96g, 1 eq) sont placés dans un réacteur. 40ml d'éther de méthyle et de tert-butyle sont ajoutés. La suspension est agitée sous reflux pendant 3 heures, puis immédiatement filtrée. Le gâteau est lavé 2 fois à l'éther de méthyle et de tert-butyle, puis séché pendant 1 heure. 5,28g d'un solide blanc correspondant au produit du titre sont obtenus.
Rendement: 66,3%
Point de fusion : 237°C

### Exemple 2 : Complexe agomélatine / acide 1,5-naphtalène disulfonique (2/1) monohydrate

### Mode-opératoire 1

L'agomélatine (5,00g, 1 eq) et l'acide 1,5-naphtalène disulfonique anhydre (5,92g, 1 eq) sont placés dans un réacteur. 10ml d'éthanol et 20ml d'eau sont ajoutés. La suspension est agitée sous reflux pendant 0,5 heure afin qu'elle devienne limpide. Le milieu est ensuite refroidi naturellement sous agitation pendant 0,5 heure, et la suspension est filtrée. Le gâteau est lavé avec de l'éthanol et de l'eau, puis séché pendant 1 heure. 5,15g d'un solide blanc sont obtenus.
Rendement: 63,2%
Point de fusion : 116°C (endotherme de déshydratation), 238°C

### Mode-opératoire 2

L'agomélatine (5,00g, 1 eq) et l'acide 1,5-naphtalène disulfonique tétrahydrate (7,40g, 1 eq) sont introduits dans un réacteur. 10ml d'éthanol et 20ml d'eau sont ajoutés. La suspension est agitée sous reflux pendant 0,5 heure afin qu'elle devienne limpide. Le milieu est ensuite refroidi naturellement sous agitation pendant 0,5 heure, et la suspension est filtrée. Le gâteau est lavé avec de l'éthanol et de l'eau, puis séché pendant 1 heure. 4,90g d'un solide blanc sont obtenus.
Rendement : 60,2%
Point de fusion : 116°C (endotherme de déshydratation), 238°C

### Mode-opératoire 3

L'agomélatine (0,5g) et l'acide 1,5-naphtalène disulfonique tétrahydrate (0,370g) sont placés dans une jarre de 50ml non oxydable. Deux billes en acier inox de 12 mm de diamètre sont ajoutées et la jarre est fermée. Des vibrations ayant une fréquence de 30 Hz sont appliquées pendant 15 minutes pour conduire, après une nuit de séchage à température ambiante, à 0,805g de solide.
Point de fusion : 116°C (endotherme de déshydratation), 238°C

### Mode-opératoire 4

L'agomélatine (0,5g) et l'acide 1,5-naphtalène disulfonique tétrahydrate (0,370g) sont placés dans une jarre de 50ml non oxydable. Deux billes en acier inox de 12 mm de diamètre sont ajoutées et la jarre est fermée. 100µl d'éther de méthyle et de tert-butyle sont ajoutés. Des vibrations ayant une fréquence de 30 Hz sont appliquées pendant 30 minutes pour conduire, après une nuit de séchage à température ambiante, à 0,803g de solide.
Point de fusion : 116°C (endotherme de déshydratation), 238°C

### Exemple 3 : Complexe agomélatine / acide benzène sulfonique (2/1)

L'agomélatine (5,00g, 2 eq) et l'acide benzène sulfonique (1,62g, 1 eq) sont introduits dans un réacteur. 10ml d'éthanol et 15ml (10ml + 5ml) de toluène sont ajoutés. La suspension est agitée sous reflux pendant 0,5 heure afin qu'elle devienne claire (si la solution n'est pas limpide, on rajoute de l'éthanol jusqu'à ce qu'elle le devienne). Le milieu est ensuite refroidi naturellement jusqu'à 5°C sous agitation pendant 0,5 heure, et la suspension est filtrée. Le gâteau est séché pendant 1 heure. 4,31g d'un solide blanc correspondant au produit du titre sont obtenus.
Rendement : 65,2%
Point de fusion : 116°C

Dans les exemples ci-dessus, on peut utiliser l'agomélatine disponible dans le commerce ou préparé selon une des méthodes décrites dans l'art antérieur.

### Exemple 4 : Compositions pharmaceutiques : gélules dosées à 25 mg d'agomélatine Composition pharmaceutique contenant le composé de l'Exemple 1

| Formulation pour la préparation de 1000 gélules contenant chacune 25 mg d'agomélatine | |
|---|---|
| Composé de l'Exemple 1 | 39,8 g |
| Lactose (Spherolac 100) | 85,2 g |
| Amidon 1500 | 25,5 g |
| CMS-Na | 8,5 g |
| Ac-Di-Sol ® (FMC) | 17 g |
| Acide Stéarique | 3,4 g |

### Composition pharmaceutique contenant le composé de l'Exemple 2

| Formulation pour la préparation de 1000 gélules contenant chacune 25 mg d'agomélatine | |
|---|---|
| Composé de l'Exemple 2 | 40,7 g |
| Lactose (Spherolac 100) | 85,2 g |
| Amidon 1500 | 25,5 g |
| CMS-Na | 8,5 g |
| Ac-Di-Sol ® (FMC) | 17 g |
| Acide Stéarique | 3,4 g |

### Composition pharmaceutique contenant le composé de l'Exemple 3

| Formulation pour la préparation de 1000 gélules contenant chacune 25 mg d'agomélatine | |
|---|---|
| Composé de l'Exemple 3 | 33,1 g |
| Lactose (Spherolac 100) | 85,2 g |
| Amidon 1500 | 25,5 g |
| CMS-Na | 8,5 g |
| Ac-Di-Sol ® (FMC) | 17 g |
| Acide Stéarique | 3,4 g |

### Exemple 5 : Compositions pharmaceutiques : comprimés dosés à 25 mg d'agomélatine

Formulation pour la préparation de 1000 comprimés contenant chacun 25 mg d'agomélatine:

| | |
|---|---|
| Composé de l'Exemple 1 ........................................................................................ | 39,8 g |
| Lactose monohydrate .............................................................................................. | 115 g |
| Stéarate de Magnésium .......................................................................................... | 2 g |
| Amidon de maïs...................................................................................................... | 33 g |
| Maltodextrines......................................................................................................... | 15 g |
| Silice colloïdale anhydre ........................................................................................ | 1 g |
| Amidon de maïs prégélatinisé, Type A................................................................... | 9 g |

Formulation pour la préparation de 1000 comprimés contenant chacun 25 mg d'agomélatine:

| | |
|---|---|
| Composé de l'Exemple 2................................................................................................. | 40,7 g |
| Lactose monohydrate .............................................................................................. | 115 g |
| Stéarate de Magnésium .......................................................................................... | 2 g |
| Amidon de maïs ............................................................................................ | 33 g |
| Maltodextrins .................................................................................... | 15 g |
| Silice colloïdale anhydre ......................................................................................... | 1 g |
| Amidon de maïs prégélatinisé, Type A................................................................... | 9 g |

Formulation pour la préparation de 1000 comprimés contenant chacun 25 mg d'agomélatine:

| | |
|---|---|
| Composé de l'Exemple 3................................................................................................. | 33,1 g |
| Lactose monohydrate .............................................................................................. | 115 g |
| Stéarate de Magnésium ........................................................................................... | 2 g |
| Amidon de maïs ...................................................................................................... | 33 g |
| Maltodextrins.......................................................................................................... | 15 g |
| Silice colloïdale anhydre ......................................................................................... | 1 g |
| Amidon de maïs prégélatinisé, Type A................................................................... | 9 g |

### Méthodes de détection et Résultats

### 1. Pureté des échantillons

Conditions chromatographiques : colonne C18 ; phase mobile : tampon phosphate 10mmol/L (ajusté à pH 7,0 avec NaOH) : acétonitrile 2:7 (v/v) ; température de la colonne : 40°C ; longueur d'onde de détection : 220 nm ; méthode standard interne utilisée avec le composé de l'Exemple 1.

Des solutions à 1 mg/ml des composés de l'invention sont préparées avec la phase mobile. 10 µl de chaque solution sont injectés dans le système de chromatographie liquide et les chromatogrammes sont enregistrés.

Les composés de l'invention ont tous des puretés supérieures ou égales à 99%.

### 2. Stabilité

Des échantillons des composés des Exemples 1, 2 et 3 sont placés dans des incubateurs dans des conditions dénaturantes et la stabilité est déterminées par des mesures de DSC sur 2 mois. Les résultats sont présentés dans le tableau 4 :

**Tableau 4**

| | 25°C, 60%HR BO | 50°C BF | 70°C BF |
|---|---|---|---|
| Composé de l'Exemple 1 | stable | stable | stable |
| Composé de l'Exemple 2 | stable | stable | stable |
| Composé de l'Exemple 3 | stable | stable | stable |

| | | | |
|---|---|---|---|
| HR = Humidité relative; BO = Bouteille Ouverte; BF = Bouteille Fermée | | | |

Les composes de l'invention sont stables dans des conditions fortement dénaturantes, ce qui est favorable à leur utilisation dans des compositions pharmaceutiques.

### 3. Solubilité

A l'aide d'une méthode standard externe, les composés des Exemples 1, 2 et 3 sont testés en HPLC, et comparés avec l'agomélatine de forme II. Les résultats sont présentés dans le tableau 5 sous forme de % d'accroissement de la solubilité par rapport à la solubilité de l'agomélatine de forme II :

**Tableau 5**

| Echantillon | Solubilité (accroissement versus Agomélatine forme II) | | |
|---|---|---|---|
| | Dans l'eau | Dans HCl 0,1N | Dans un tampon pH6,8 |
| Composé de l'Exemple 1 | +18% | +25% | +48% |
| Composé de l'Exemple 2 | +12% | +75% | +57% |
| Composé de l'Exemple 3 | +22% | +32% | +46% |

Les résultats montrent que les complexes d'agomélatine et d'acides sulfoniques de la présente invention ont une solubilité supérieure à l'agomélatine de forme II *per se* dans l'eau, dans HCl 0,1N, semblable aux fluides gastriques humains, ou dans un tampon à pH 6,8. Ces résultats montrent que les complexes ont un bien meilleur potentiel en terme de biodisponibilité que l'agomélatine de forme II.

### 4. Analyses DSC

Environ 5-10 mg des composés des Exemples 1, 2 et 3 sont pesés dans un creuset en aluminium fermé avec un couvercle en aluminium percé (non hermétique), sauf précision contraire. L'échantillon est introduit dans un appareil TA Q1000 (équipé avec un refroidisseur), refroidi et maintenu à 25°C. Après stabilisation thermique, l'échantillon et la référence sont chauffés de 200°C à 250°C à une vitesse de 10°C/min et la réponse au flux thermique est enregistrée. L'azote est utilisé comme gaz de purge, à un débit de 100 cm³/min.

Les thermogrammes de DSC obtenus avec les composés des Exemples 1, 2 et 3 sont rapportés dans les Figures 2, 4 et 6.

### 5. Analyse de la structure cristalline

Les conditions de mesure des diagrammes de diffraction X sur poudre des produits des Exemples 1, 2 et 3 sont les suivantes :
Environ 50mg des composés des Exemples 1, 2 et 3 sont placés entre deux films Kapton® et fixé sur le support d'échantillons. L'échantillon est ensuite placé dans un diffractomètre PANALYTICAL XPERT-PRO MPD en mode transmission dans les conditions suivantes :
Paramètres du générateur: 45 kV / 40 mA,
Configuration theta/theta
Anode : Cu
K-Alphal [Å] 1,54060
K-Alpha2 [Å] 1,54443
K-Beta [Å] 1,39225
K-A2 / K-A1 Ratio 0,50000
Mode de balayage : continu de 3° à 55° (angle de Bragg 2 thêta) Pas [°2Th.] 0,0170
Durée du pas [s] 35,5301
Angle de départ [°2Th.] 3,0034
Angle de fin [°2Th.] 54,9894
Rotation: oui

Les diagrammes de diffraction X sur poudre obtenus pour les Exemples 1, 2 et 3 sont représentés dans les Figures 1, 3 et 5.

## Revendications

1. Complexes d'agomélatine et d'acides sulfoniques de formule (I) : 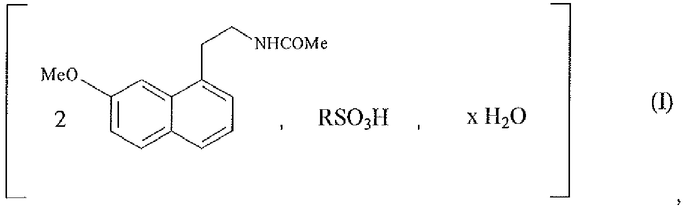 dans laquelle x représente 0 ou 1, et RSO₃H représente l'acide 1,5-naphtalène disulfonique ou l'acide benzène sulfonique.

2. Complexe d'agomélatine de formule (I) selon la revendication 1 qui est le complexe agomélatine / acide 1,5-naphtalène disulfonique (2/1).

3. Complexe d'agomélatine de formule (I) selon la revendication 2 **caractérisé par** son diagramme de diffraction X sur poudre exprimé en termes de distance inter-réticulaire d, d'angle de Bragg 2 thêta (exprimés en °±0,2), et d'intensité relative suivant:
| 2-Theta (°) exp. | d (Å) exp. | Intensity (%) |
|---|---|---|
| 6.3716 | 13.87229 | 18.97 |
| 11.3804 | 7.77552 | 17.98 |
| 11.9227 | 7.42299 | 36.06 |
| 12.5064 | 7.07784 | 100.00 |
| 12.6590 | 6.99288 | 13.75 |
| 14.5508 | 6.08767 | 44.17 |
| 15.5658 | 5.69292 | 11.96 |
| 16.2029 | 5.47051 | 42.63 |
| 16.9421 | 5.23346 | 25.85 |
| 17.6267 | 5.03171 | 18.67 |
| 19.4300 | 4.56857 | 49.04 |
| 20.2146 | 4.39301 | 22.77 |
| 21.4353 | 4.14550 | 17.80 |
| 21.6713 | 4.10090 | 22.84 |
| 22.2180 | 4.00121 | 64.19 |
| 22.4174 | 3.96607 | 10.83 |
| 24.0749 | 3.69664 | 29.61 |
| 24.5048 | 3.63275 | 13.33 |
| 25.1744 | 3.53763 | 20.58 |
| 25.7599 | 3.45853 | 23.59 |
incluant les formes dont les angles de diffraction correspondent à ±0.2° près.

4. Complexe d'agomélatine de formule (I) selon la revendication 1 qui est le complexe agomélatine / acide 1,5-naphthalène disulfonique (2/1) monohydrate.

5. Complexe d'agomélatine de formule (I) selon la revendication 4 **caractérisé par** son diagramme de diffraction X sur poudre exprimé en termes de distance inter-réticulaire d, d'angle de Bragg 2 thêta (exprimés en °±0,2), et d'intensité relative suivant:
| 2-Theta (°) exp. | d (Å) exp. | Intensity (%) |
|---|---|---|
| 9.6680 | 9.14852 | 12.45 |
| 12.4885 | 7.08796 | 57.23 |
| 12.6164 | 7.01639 | 28.61 |
| 14.5042 | 6.10715 | 57.42 |
| 16.2684 | 5.44863 | 25.67 |
| 16.4624 | 5.38484 | 32.93 |
| 16.8967 | 5.24739 | 90.90 |
| 19.3772 | 4.58091 | 10.50 |
| 22.4767 | 3.95573 | 100.00 |
| 23.4111 | 3.79992 | 20.49 |
| 23.5330 | 3.78051 | 44.02 |
| 23.6735 | 3.75840 | 21.92 |
| 24.0477 | 3.70076 | 13.67 |
| 24.5716 | 3.62303 | 13.43 |
| 25.1240 | 3.54460 | 12.46 |
| 26.6602 | 3.34374 | 19.10 |
| 28.1333 | 3.16930 | 12.07 |
| 28.2443 | 3.15971 | 22.49 |
incluant les formes dont les angles de diffraction correspondent à ±0.2° près.

6. Complexe d'agomélatine de formule (I) selon la revendication 1 qui est le complexe agomélatine / acide benzène sulfonique (2/1).

7. Complexe d'agomélatine de formule (1) selon la revendication 6 **caractérisé par** son diagramme de diffraction X sur poudre exprimé en termes de distance inter-réticulaire d, d'angle de Bragg 2 thêta (exprimés en °±0,2), et d'intensité relative suivant:
| 2-Theta (°) exp. | d (Å) exp. | Intensity (%) |
|---|---|---|
| 8.0711 | 10.95469 | 35.25 |
| 12.6820 | 6.98026 | 68.37 |
| 12.7706 | 6.93203 | 65.37 |
| 13.0114 | 6.80427 | 15.18 |
| 13.3054 | 6.65458 | 31.84 |
| 14.9475 | 5.92700 | 19.42 |
| 15.1121 | 5.86283 | 70.19 |
| 15.4873 | 5.72160 | 14.16 |
| 16.1644 | 5.48344 | 12.98 |
| 17.2360 | 5.14486 | 21.06 |
| 18.1046 | 4.89993 | 36.33 |
| 18.6255 | 4.76406 | 10.91 |
| 18.8009 | 4.72001 | 33.43 |
| 20.0908 | 4.41978 | 30.26 |
| 20.4742 | 4.33788 | 42.37 |
| 20.6921 | 4.29270 | 56.78 |
| 20.8640 | 4.25771 | 26.42 |
| 21.7142 | 4.09289 | 13.88 |
| 23.3683 | 3.80679 | 15.16 |
| 23.6410 | 3.76349 | 100.00 |
| 24.9314 | 3.57154 | 26.81 |
| 25.6543 | 3.47253 | 10.71 |
| 27.5599 | 3.23660 | 14.00 |
incluant les formes dont les angles de diffraction correspondent à ±0.2° près.

8. Procédé d'obtention des complexes d'agomélatine et d'acides sulfoniques selon l'une des revendications 1 à 7 **caractérisé en ce que** :
- l'agomélatine et les acides sulfoniques sont mélangés au sein d'un solvant organique ou hydro-organique aqueux dans les proportions désirées;
- la solution obtenue est agitée et optionnellement chauffée à une température au plus égale à la température d'ébullition du solvant choisi ;
- le milieu est refroidi sous agitation et le co-cristal précipite naturellement ou précipite après reprise dans un deuxième solvant ;
- le précipité obtenu est filtré et séché.

9. Procédé de préparation des complexes d'agomélatine et d'acides sulfoniques selon l'une des revendications 1 à 7 **caractérisé en ce que** les deux constituants sont co-broyés.

10. Procédé de préparation des complexes d'agomélatine et d'acides sulfoniques selon l'une des revendications 1 à 7 **caractérisé en ce que** les deux constituants sont mélangés au sein d'un solvant organique ou hydro-organique puis congelés et séchés à très basse température.

11. Procédé de préparation des complexes d'agomélatine et d'acides sulfoniques selon l'une des revendications 1 à 7 **caractérisé en ce que** les poudres d'agomélatine et de l'acide considéré sont mélangées dans un mélangeur, puis le mélange est extrudé par extrusion bi-vis sans filière pour obtenir un grain solide directement en sortie d'extrudeuse.

12. Compositions pharmaceutiques contenant comme principe actif un des complexes d'agomélatine et d'acides sulfoniques selon l'une des revendications 1 à 7, en combinaison avec un ou plusieurs véhicules inertes, non toxiques et pharmaceutiquement acceptables.

13. Utilisation de compositions pharmaceutiques selon la revendication 12 pour la fabrication de médicaments pour traiter les troubles du système mélatoninergique.

14. Utilisation de compositions pharmaceutiques selon la revendication 12 pour la fabrication de médicaments pour le traitement du stress, des troubles du sommeil, des troubles de l'anxiété et notamment du trouble anxiété généralisée, des troubles obsessionnels compulsifs, des troubles de l'humeur et notamment des troubles bipolaires, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale ainsi que dans les dysfonctionnements sexuels, en tant qu'inhibiteurs de l'ovulation, d'immunomodulateurs et dans le traitement des cancers.

15. Complexes d'agomélatine et d'acides sulfoniques de formule (I) selon l'une des revendications 1 à 7 pour le traitement des troubles du système mélatoninergique.

16. Complexes d'agomélatine et d'acides sulfoniques de formule (I) selon l'une des revendications 1 à 7 pour le traitement du stress, des troubles du sommeil, des troubles de l'anxiété et notamment du trouble anxiété généralisée, des troubles obsessionnels compulsifs, des troubles de l'humeur et notamment des troubles bipolaires, de la dépression majeure, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, de la douleur, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale ainsi que dans les dysfonctionnements sexuels, en tant qu'inhibiteurs de l'ovulation, d'immunomodulateurs et dans le traitement des cancers.

## Patentansprüche

1. Komplexe aus Agomelatin und Sulfonsäuren der Formel (I): 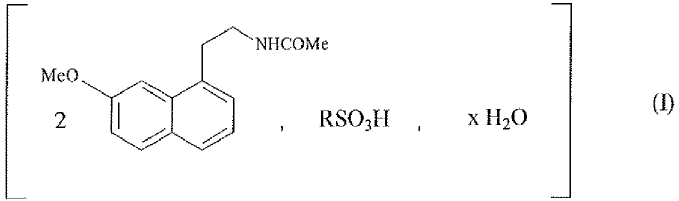 in der x 0 oder 1 bedeutet und RSO₃H 1,5-Naphthalin-disulfonsäure oder Benzolsulfonsäure bedeutet.

2. Agomelatinkomplex der Formel (I) nach Anspruch 1, nämlich der Agomelatin / 1,5-Naphthalin-disulfonsäure-Komplex (2/1).

3. Agomelatinkomplex der Formel (I) nach Anspruch 1, **gekennzeichnet durch** sein folgendes Pulverröntgenbeugungsdiagramm angegeben als Netzebenenabstand d, Bragg-2-Theta-Winkel (angegeben in ° ± 0,2) und relativer Intensität:
| 2-Theta (°) exp. | d (Å) exp. | Intensität (%) |
|---|---|---|
| 6,3716 | 13,87229 | 18,97 |
| 11,3804 | 7,77552 | 17,98 |
| 11,9227 | 7,42299 | 36,06 |
| 12,5064 | 7,07784 | 100,00 |
| 12,6590 | 6,99288 | 13,75 |
| 14,5508 | 6,08767 | 44,17 |
| 15,5658 | 5,69292 | 11,96 |
| 16,2029 | 5,47051 | 42,63 |
| 16,9421 | 5,23346 | 25,85 |
| 17,6267 | 5,03171 | 18,67 |
| 19,4300 | 4,56857 | 49,04 |
| 20,2146 | 4,39301 | 22,77 |
| 21,4353 | 4,14550 | 17,80 |
| 21,6713 | 4,10090 | 22,84 |
| 22,2180 | 4,00121 | 64,19 |
| 22,4174 | 3,96607 | 10,83 |
| 24,0749 | 3,69664 | 29,61 |
| 24,5048 | 3,63275 | 13,33 |
| 25,1744 | 3,53763 | 20,58 |
| 25,7599 | 3,45853 | 23,59 |
einschließlich der Formen, deren Beugungswinkel auch ± 0,2° entsprechen.

4. Agomelatinkomplex der Formel (I) nach Anspruch 1, nämlich Agomelatin / 1,5-Naphthalin-disulfonsäure-(2/1)-Monohydrat-Komplex.

5. Agomelatinkomplex der Formel (I) nach Anspruch 4, **gekennzeichnet durch** sein folgendes Pulverröntgenbeugungsdiagramm angegeben als Netzebenenabstand d, Bragg-2-Theta-Winkel (angegeben in ° ± 0,2) und relativer Intensität:
| 2-Theta (°) exp. | d (Å) exp. | Intensität (%) |
|---|---|---|
| 9,6680 | 9,14852 | 12,45 |
| 12,4885 | 7,08796 | 57,23 |
| 12,6164 | 7,01639 | 28,61 |
| 14,5042 | 6,10715 | 57,42 |
| 16,2684 | 5,44863 | 25,67 |
| 16,4624 | 5,38484 | 32,93 |
| 16,8967 | 5,24739 | 90,90 |
| 19,3772 | 4,58091 | 10,50 |
| 22,4767 | 3,95573 | 100,00 |
| 23,4111 | 3,79992 | 20,49 |
| 23,5330 | 3,78051 | 44,02 |
| 23,6735 | 3,75840 | 21,92 |
| 24,0477 | 3,70076 | 13,67 |
| 24,5716 | 3,62303 | 13,43 |
| 25,1240 | 3,54460 | 12,46 |
| 26,6602 | 3,34374 | 19,10 |
| 28,1333 | 3,16930 | 12,07 |
| 28,2443 | 3,15971 | 22,49 |
einschließlich der Formen, deren Beugungswinkel auch ± 0,2° entsprechen.

6. Agomelatinkomplex der Formel (I) nach Anspruch 1, nämlich Agomelatin / Benzolsulfonsäure-(2/1)-Komplex.

7. Agomelatinkomplex der Formel (I) nach Anspruch 6, **gekennzeichnet durch** sein folgendes Pulverröntgenbeugungsdiagramm angegeben als Netzebenenabstand d, Bragg-2-Theta-Winkel (angegeben in ° ± 0,2) und relativer Intensität:
| 2-Theta (°) exp. | d (Å) exp. | Intensität (%) |
|---|---|---|
| 8,0711 | 10,95469 | 35,25 |
| 12,6820 | 6,98026 | 68,37 |
| 12,7706 | 6,93203 | 65,37 |
| 13,0114 | 6,80427 | 15,18 |
| 13,3054 | 6,65458 | 31,84 |
| 14,9475 | 5,92700 | 19,42 |
| 15,1121 | 5,86283 | 70,19 |
| 15,4873 | 5,72160 | 14,16 |
| 16,1644 | 5,48344 | 12,98 |
| 17,2360 | 5,14486 | 21,06 |
| 18,1046 | 4,89993 | 36,33 |
| 18,6255 | 4,76406 | 10,91 |
| 18,8009 | 4,72001 | 33,43 |
| 20,0908 | 4,41978 | 30,26 |
| 20,4742 | 4,33788 | 42,37 |
| 20,6921 | 4,29270 | 56,78 |
| 20,8640 | 4,25771 | 26,42 |
| 21,7142 | 4,09289 | 13,88 |
| 23,3683 | 3,80679 | 15,16 |
| 23,6410 | 3,76349 | 100,00 |
| 24,9314 | 3,57154 | 26,81 |
| 25,6543 | 3,47253 | 10,71 |
| 27,5599 | 3,23660 | 14,00 |
einschließlich der Formen, deren Beugungswinkel auch ± 0,2° entsprechen.

8. Verfahren zur Herstellung der Komplexe aus Agomelatin und Sulfonsäuren gemäß einem der Ansprüche 1 bis 7, **dadurch gekennzeichnet, dass** :
- Agomelatin und die Sulfonsäuren in einem organischen oder wässrig-organischen Lösungsmittel in den gewünschten Mengenverhältnissen vermischt werden;
- die erhaltene Lösung gerührt und gegebenenfalls auf eine Temperatur, die höchstens gleich ist der Siedetemperatur des gewählten Lösungsmittels, erhitzt wird;
- das Medium unter Rühren abgekühlt wird und der Co-Kristall natürlich ausfällt oder nach der Aufnahme in ein zweites Lösungsmittel ausfällt;
- der erhaltene Niederschlag filtriert und getrocknet wird.

9. Verfahren zur Herstellung der Komplexe aus Agomelatin und Sulfonsäuren nach einem der Ansprüche 1 bis 7, **dadurch gekennzeichnet, dass** die beiden Bestandteile gemeinsam vermahlen werden.

10. Verfahren zur Herstellung der Komplexe aus Agomelatin und Sulfonsäuren nach einem der Ansprüche 1 bis 7, **dadurch gekennzeichnet, dass** die beiden Bestandteile in einem organischen oder wässrig-organischen Lösungsmittel vermischt werden und dann eingefroren und bei sehr niedriger Temperatur getrocknet werden.

11. Verfahren zur Herstellung der Komplexe aus Agomelatin und Sulfonsäuren gemäß einem der Ansprüche 1 bis 7, **dadurch gekennzeichnet, dass** die Pulver von Agomelatin und der betreffenden Säure in einem Mischer vermischt werden und die Mischung dann durch Doppelschneckenextrusion ohne Extrusionsdüse extrudiert wird, um direkt am Ausgang des Extruders ein festes Korn zu erhalten.

12. Pharmazeutische Zubereitung enthaltend als Wirkstoff einen der Komplexe aus Agomelatin und Sulfonsäuren gemäß einem der Ansprüche 1 bis 7 in Kombination mit einem oder mehreren inerten, nichttoxischen und pharmazeutisch annehmbaren Hilfsstoffen.

13. Verwendung der pharmazeutischen Zubereitung nach Anspruch 12 zur Herstellung von Arzneimitteln zur Behandlung von Störungen des melatoninergischen Systems.

14. Verwendung der pharmazeutischen Zubereitung nach Anspruch 12 zur Herstellung von Arzneimitteln zur Behandlung von Stress, Schlafstörungen, Angststörungen und insbesondere allgemeinen Angststörungen, kompulsiven Zwangsstörungen, Gemütsstörungen und insbesondere bipolaren Störungen, Major-Depression, saisonal bedingten Depressionen, kardiovaskulären Erkrankungen, Erkrankungen des Verdauungssystems, Schlaflosigkeit und Müdigkeit als Folge von Zeitverschiebungen, Schizophrenie, Panikanfällen, Melancholie, Appetitstörungen, Fettsucht, Schlaflosigkeit, Schmerz, psychotischen Störungen, Epilepsie, Diabetes, der Parkinsonschen Krankheit, der senilen Demenz, verschiedenen Störungen, die mit dem normalen oder pathologischen Altern verknüpft sind, Migräne, Gedächtnisverlusten, der Alzheimerschen Krankheit sowie Störungen der Gehirndurchblutung sowie sexuellen Dysfunktionen, als Ovulationshemmer, Immunomodulatoren und bei der Behandlung von Krebs.

15. Komplexe aus Agomelatin und Sulfonsäuren der Formel (I) nach einem der Ansprüche 1 bis 7 zur Behandlung von Störungen des melatoninergischen Systems.

16. Komplexe aus Agomelatin und Sulfonsäuren der Formel (I) nach einem der Ansprüche 1 bis 7 zur Behandlung von Stress, Schlafstörungen, Angststörungen und insbesondere allgemeinen Angststörungen, kompulsiven Zwangsstörungen, Gemütsstörungen und insbesondere bipolaren Störungen, Major-Depression, saisonal bedingten Depressionen, kardiovaskulären Erkrankungen, Erkrankungen des Verdauungssystems, Schlaflosigkeit und Müdigkeit als Folge von Zeitverschiebungen, Schizophrenie, Panikanfällen, Melancholie, Appetitstörungen, Fettsucht, Schlaflosigkeit, Schmerz, psychotischen Störungen, Epilepsie, Diabetes, der Parkinsonschen Krankheit, der senilen Demenz, verschiedenen Störungen, die mit dem normalen oder pathologischen Altern verknüpft sind, Migräne, Gedächtnisverlusten, der Alzheimerschen Krankheit sowie Störungen der Gehirndurchblutung sowie sexuellen Dysfunktionen, als Ovulationshemmer, Immunomodulatoren und bei der Behandlung von Krebs.

## Claims

1. Complexes of agomelatine and sulphonic acids of formula (I): 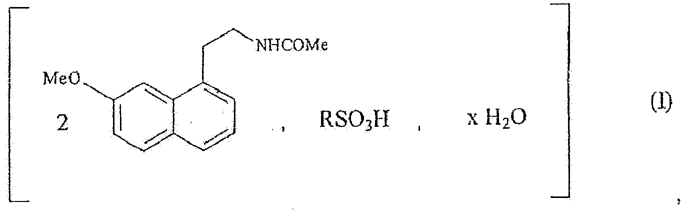 wherein x represents 0 or 1, and RSO₃H represents 1,5-naphthalenedisulphonic acid or benzenesulphonic acid.

2. Complex of agomelatine of formula (I) according to claim 1 which is the complex agomelatine/1,5-naphthalenedisulphonic acid (2/1).

3. Complex of agomelatine of formula (I) according to claim 2, **characterised by** its X-ray powder diffraction diagram expressed in terms of interplanar distance d, Bragg's angle 2 theta (expressed in °±0.2), and relative intensity, as follows:
| 2-Theta (°) exp. | d (Å) exp. | Intensity (%) |
|---|---|---|
| 6.3716 | 13.87229 | 18.97 |
| 11.3804 | 7.77552 | 17.98 |
| 11.9227 | 7.42299 | 36.06 |
| 12.5064 | 7.07784 | 100.00 |
| 12.6590 | 6.99288 | 13.75 |
| 14.5508 | 6.08767 | 44.17 |
| 15.5658 | 5.69292 | 11.96 |
| 16.2029 | 5.47051 | 42.63 |
| 16.9421 | 5.23346 | 25.85 |
| 17.6267 | 5.03171 | 18.67 |
| 19.4300 | 4.56857 | 49.04 |
| 20.2146 | 4.39301 | 22.77 |
| 21.4353 | 4.14550 | 17.80 |
| 21.6713 | 4.10090 | 22.84 |
| 22.2180 | 4.00121 | 64.19 |
| 22.4174 | 3.96607 | 10.83 |
| 24.0749 | 3.69664 | 29.61 |
| 24.5048 | 3.63275 | 13.33 |
| 25.1744 | 3.53763 | 20.58 |
| 25.7599 | 3.45853 | 23.59 |
including the forms whose diffraction angles correspond to within ±0.2°.

4. Complex of agomelatine of formula (I) according to claim 1 which is the complex agomelatine/1,5-naphthalenedisulphonic acid (2/1) monohydrate.

5. Complex of agomelatine of formula (I) according to claim 4, **characterised by** its X-ray powder diffraction diagram expressed in terms of interplanar distance d, Bragg's angle 2 theta (expressed in °±0.2), and relative intensity, as follows:
| 2-Theta (°) exp. | d (Å) exp. | Intensity (%) |
|---|---|---|
| 9.6680 | 9.14852 | 12.45 |
| 12.4885 | 7.08796 | 57.23 |
| 12.6164 | 7.01639 | 28.61 |
| 14.5042 | 6.10715 | 57.42 |
| 16.2684 | 5.44863 | 25.67 |
| 16.4624 | 5.38484 | 32.93 |
| 16.8967 | 5.24739 | 90.90 |
| 19.3772 | 4.58091 | 10.50 |
| 22.4767 | 3.95573 | 100.00 |
| 23.4111 | 3.79992 | 20.49 |
| 23.5330 | 3.78051 | 44.02 |
| 23.6735 | 3.75840 | 21.92 |
| 24.0477 | 3.70076 | 13.67 |
| 24.5716 | 3.62303 | 13.43 |
| 25.1240 | 3.54460 | 12.46 |
| 26.6602 | 3.34374 | 19.10 |
| 28.1333 | 3.16930 | 12.07 |
| 28.2443 | 3.15971 | 22.49 |
including the forms whose diffraction angles correspond to within ±0.2°.

6. Complex of agomelatine of formula (I) according to claim 1 which is the complex agomelatine/benzenesulphonic acid (2/1).

7. Complex of agomelatine of formula (I) according to claim 6, **characterised by** its X-ray powder diffraction diagram expressed in terms of interplanar distance d, Bragg's angle 2 theta (expressed in °±0.2), and relative intensity, as follows:
| 2-Theta (°) exp. | d (Å) exp. | Intensity (%) |
|---|---|---|
| 8.0711 | 10.95469 | 35.25 |
| 12.6820 | 6.98026 | 68.37 |
| 12.7706 | 6.93203 | 65.37 |
| 13.0114 | 6.80427 | 15.18 |
| 13.3054 | 6.65458 | 31.84 |
| 14.9475 | 5.92700 | 19.42 |
| 15.1121 | 5.86283 | 70.19 |
| 15.4873 | 5.72160 | 14.16 |
| 16.1644 | 5.48344 | 12.98 |
| 17.2360 | 5.14486 | 21.06 |
| 18.1046 | 4.89993 | 36.33 |
| 18.6255 | 4.76406 | 10.91 |
| 18.8009 | 4.72001 | 33.43 |
| 20.0908 | 4.41978 | 30.26 |
| 20.4742 | 4.33788 | 42.37 |
| 20.6921 | 4.29270 | 56.78 |
| 20.8640 | 4.25771 | 26.42 |
| 21.7142 | 4.09289 | 13.88 |
| 23.3683 | 3.80679 | 15.16 |
| 23.6410 | 3.76349 | 100.00 |
| 24.9314 | 3.57154 | 26.81 |
| 25.6543 | 3.47253 | 10.71 |
| 27.5599 | 3.23660 | 14.00 |
including the forms whose diffraction angles correspond to within ±0.2°.

8. Process for obtaining the complexes of agomelatine and sulphonic acids according to any one of claims 1 to 7, **characterised in that**:
- agomelatine and the sulphonic acids are mixed in an organic or aqueous-organic solvent in the desired proportions;
- the solution obtained is stirred and optionally heated at a temperature not greater than the boiling point of the chosen solvent;
- the mixture is cooled, with stirring, and the co-crystal precipitates naturally or precipitates after being taken up in a second solvent;
- the precipitate obtained is filtered and dried.

9. Process for the preparation of the complexes of agomelatine and sulphonic acids according to any one of claims 1 to 7, **characterised in that** the two constituents are co-ground.

10. Process for the preparation of the complexes of agomelatine and sulphonic acids according to any one of claims 1 to 7, **characterised in that** the two constituents are mixed in an organic or aqueous-organic solvent and then frozen and dried at a very low temperature.

11. Process for the preparation of the complexes of agomelatine and sulphonic acids according to any one of claims 1 to 7, **characterised in that** the powders of agomelatine and the acid in question are mixed in a mixer and the mixture is then extruded by twin-screw extrusion without a die in order to obtain a solid grain directly at the outlet of the extruder.

12. Pharmaceutical compositions comprising as active ingredient one of the complexes of agomelatine and sulphonic acids according to any one of claims 1 to 7, in combination with one or more inert, non-toxic, pharmaceutically acceptable carriers.

13. Use of pharmaceutical compositions according to claim 12 in the manufacture of medicaments for treating disorders of the melatoninergic system.

14. Use of pharmaceutical compositions according to claim 12 in the manufacture of medicaments for treating stress, sleep disorders, anxiety disorders and especially generalised anxiety disorder, obsessive compulsive disorders, mood disorders and especially bipolar disorders, major depression, seasonal affective disorder, cardiovascular pathologies, pathologies of the digestive system, insomnia and fatigue due to jet-lag, schizophrenia, panic attacks, melancholia, appetite disorders, obesity, insomnia, pain, psychotic disorders, epilepsy, diabetes, Parkinson's disease, senile dementia, various disorders associated with normal or pathological ageing, migraine, memory loss, Alzheimer's disease, and also in cerebral circulation disorders, and also in sexual dysfunctions, as ovulation inhibitors and immunomodulators and in the treatment of cancers.

15. Complexes of agomelatine and sulphonic acids of formula (I) according to any one of claims 1 to 7 for treating disorders of the melatoninergic system.

16. Complexes of agomelatine and sulphonic acids of formula (I) according to any one of claims 1 to 7 for treating stress, sleep disorders, anxiety disorders and especially generalised anxiety disorder, obsessive compulsive disorders, mood disorders and especially bipolar disorders, major depression, seasonal affective disorder, cardiovascular pathologies, pathologies of the digestive system, insomnia and fatigue due to jet-lag, schizophrenia, panic attacks, melancholia, appetite disorders, obesity, insomnia, pain, psychotic disorders, epilepsy, diabetes, Parkinson's disease, senile dementia, various disorders associated with normal or pathological ageing, migraine, memory loss, Alzheimer's disease, and also in cerebral circulation disorders, and also in sexual dysfunctions, as ovulation inhibitors and immunomodulators and in the treatment of cancers.
